(19) Europäisches Patentamt
European Patent Office
Office européen des brevets

(11) Veröffentlichungsnummer: **0 069 055**
**B1**

(12) # EUROPÄISCHE PATENTSCHRIFT

(45) Veröffentlichungstag der Patentschrift: **17.12.86**

(21) Anmeldenummer: **82810233.5**

(22) Anmeldetag: **28.05.82**

(51) Int. Cl.⁴: **C 07 D 309/32**, C 07 D 311/74, C 07 D 311/94, A 01 N 43/16 // C07C69/738

(54) **Dihydropyrone, Verfahren zu ihrer Herstellung, Mittel, welche die Dihydropyrone als Wirkstoffe enthalten, und ihre Verwendung zur Bekämpfung von Unkräutern.**

(30) Priorität: **03.06.81 CH 3630/81**

(43) Veröffentlichungstag der Anmeldung: **05.01.83 Patentblatt 83/1**

(45) Bekanntmachung des Hinweises auf die Patenterteilung: **17.12.86 Patentblatt 86/51**

(84) Benannte Vertragsstaaten: **AT BE CH DE FR GB IT LI NL SE**

(56) Entgegenhaltungen: **GB-A-2 016 463**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Tobler, Hans, Dr., Baselmattweg 157, CH- 4123 Allschwil (CH)**
Erfinder: **Föry, Werner, Dr., Benkenstrasse 65, CH- 4054 Basel (CH)**

**EP 0 069 055 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft Dihydropyrone mit herbizider Wirkung. Verfahren zu ihrer Herstellung Mittel, welche die Dihydropyrone als Wirkstoffe enthalten, und ihre Verwendung zur Bekämpfung von Unkräutern.

Aus GB-A- 2 016 463 ist es bekannt, dass 2-Phenyl-5,6-dihydro-4-pyrone über selektive herbizide Eigenschaften verfügen. Es wurde nun gefunden, dass 2-(4-Phenoxy)-phenyl-5,6-di-hydro-4-pyrone als Herbizide eine wesentlich verbesserte Selektivität gegenüber diesem Stand der Technik aufweisen.

Die neuen Dihydropyrone entsprechen der Formel (I)

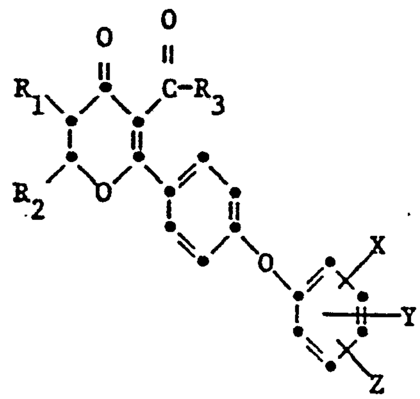

(I),

worin $R_1$ und $R_2$ unabhängig voneinander eine $C_1$-$C_4$-Alkylgruppe oder eine der beiden Substituenten auch Wasserstoff, oder $R_1$ und $R_2$ gemeinsam eine $C_2$-$C_6$-Alkylenbrücke, $R_3$ $C_1$-$C_4$-Alkoxy, Allyloxy, Propargyloxy, 2,2,2-Trichloräthoxy, 2,2,2-Trifluoräthoxy, 2-Chloräthoxy, $C_1$-$C_4$ Alkoxy. $C_1$-$C_4$ alkoxy mit maximal 5 Kohlenstofatomen, Hydroxy oder Salze dieser Carbonsäuren und X, Y und Z unabhängig voneinander Wasserstoff, Halogen, $C_1$-$C_4$-Alkyl, $C_1$-$C_4$-Alkoxy, $C_1$-$C_4$-Halogenalkyl, Trifluormethoxy, Difluormethoxy, 1,1,2,2-Tetrafluoräthoxy, -$S(O)_n$-$C_1$-$C_4$-Alkyl, Triflourmethylthio, Diflourmethylthio, Triflourmethysulfinyl wobei n für 0, 1 oder 2 steht, $C(O)OR_4$, wobei $R_4$ für Wasserstoff oder $C_1$-$C_4$-Alkyl steht, $NO_2$, CN oder $NR_5R_6$, wobei $R_5$ und $R_6$ unabhängig voneinander für Wasserstoff, $C_1$-$C_4$-Alkyl, $C_1$-$C_4$-Alkylcarbonyl oder $C_1$-$C_4$-Halogenalkylcarbonyl stehen, bedeuten, unter Einschluss ihrer Säureadditionssalze.

Unter $C_1$-$C_4$-Alkyl als Substituent oder Teil eines Substituenten ist n-Propyl Isopropyl n-Butyl sek.-Butyl tert.-Butyl, Isobutyl und insbesondere Methyl und Aethyl zu verstehen.

Eine von $R_1$ und $R_2$ gebildete Alkylenbrücke kann eine geradkettigen oder verzweigten Rest darstellen insbesondere eine $-(CH_2)_3-$ oder $-(CH_2)_4-$ Gruppe.

Unter Halogen als Substituent oder Teil eines Substituenten sind Fluor-, Chlor-, Brom- und Jodatome zu verstehen. Halogenalkylreste können ein- oder mehrfach durch die vorgenannten Halogenatome substituiert sein, wie beispielsweise $CH_2J$, $CCl_3$, $CHCl_2$, $CH_2Br$ und insbesondere $CF_3$, $CHF_2$, $CH_2CH_2Cl$, $CF_2CHF_2$, $CH_2CF_3$ und $CH_2CCl_3$.

Als Salze kommen insbesondere Metallsalze und Salze mit quaternären Ammoniumbasen oder organischen Stickstoffbasen in Betracht. Zur Salzbildung geeignete Metalle sind beispielsweise Erdalkalimetalle, wie Magnesium oder Calcium, vor allem aber die Alkalimetalle, wie Lithium, Kalium oder Natrium. Ferner sind als Salzbildner auch Uebergangsmetalle wie beispielsweise Eisen, Nickel, Kobalt, Kupfer, Zink, Chrom oder Mangan geeignet.

Beispiele für quaternäre Ammoniumbasen sind das Ammoniumkation, Tetraalkylammoniumkationen, in denen die Alkylreste unabhängig voneinander geradkettige oder verzweigte $C_1$-$C_6$-Alkylgruppen sind, wie das Tetramethylammoniumkation, das Tetraäthylammoniumkation oder das Trimethyläthylammoniumkation sowie weiterhin das Trimethylbenzylammoniumkation, das Triäthylbenzylammoniumkation das Trimethyl-2-hydroxyäthylammoniumkation und das Trimethyl-2-chloräthylammoniumkation.

Beispiele für zur Salzbildung geeignete organische Stickstoffbasen sind primäre, sekundäre und tertiäre, aliphatische und aromatische, gegebenenfalls am Kohlenwasserstoffrest hydroxylierte Amine, wie Methylamin, Aethylamin, Propylamin, Isopropylamin, die vier isomeren Butylamine, Dimethylamin, Diäthylamin, Di-n-propylamin, Di-isopropylamin, Di-n-butylamin, Pyrrolidin, Piperidin, Morpholin, Trimethylamin, Triäthylamin, Tri-n-propylamin, Chinuclidin, Pyridin, Chinolin, Isochinolin sowie Methanolamin, Aethanolamin, Propanolamin, Dimethanolamin, Diäthanolamin oder Triäthanolamin.

Verbindungen der Formel (I), in denen $R_1$ und $R_2$ Alkylgruppen bedeuten, können in Form von zwei Diastereomeren vorliegen, von denen das eine eine cis-, das andere eine trans-Konfiguration aufweist. Aus Isomerengemischen von Verbindungen der Formel (I) lassen sich die Isomere auf an sich bekannte Weise, beispielsweise säulenchromatografisch, trennen. Die vorliegende Erfindung umfasst cis-Isomere, trans-Isomere und Isomerengemische von Verbindungen der Formel (I).

Bevorzugt sind Verbindungen der Formel (I), worin $R_1$ für $C_1$-$C_4$-Alkyl und $R_2$ für Methyl oder Aethyl oder einer der Substituenten $R_1$ und $R_2$ auch für Wasserstoff steht oder $R_1$ und $R_2$ zusammen eine $-(CH_2)_3-$ oder $-(CH_2)_4-$ Gruppe bilden $R_3$ für $C_1$-$C_4$-Alkoxy, welches geradkettig oder verzweigt sein kann, Methoxymethoxy oder 2-(Methoxy)-äthoxy steht, einer der Substituenten X, Y und Z für Wasserstoff steht und die beiden anderen Substituenten unabhängig voneinander Wasserstoff, Chlor, Brom, Jod, Fluor, Methyl, Aethyl, Methoxy, Trifluormethyl, Trifluormethoxy, Difluormethoxy, 1,1,2,2-Tetrafluoräthoxy, Methylthio, Methylsulfinyl, Methylsulfonyl, Trifluormethylthio, Difluormethylthio, Trifluormethylsulfinyl,- Methoxycarbonyl, Aethoxycarbonyl, Nitro, Cyano, Amino, Methylamino oder Dimethylamino bedeuten.

Besonders bevorzugt sind Verbindungen der Formel I, worin einer der Substituenten $R_1$ und $R_2$ für Wasserstoff und der andere für Methyl oder Aethyl oder $R_1$ und $R_2$ beide für Methyl stehen, $R_3$ eine Methoxy- oder Aethoxygruppe darstellt, einer der Substituenten X. Y und Z für Wasserstoff steht und die beiden anderen Substituenten unabhängig voneinander Wasserstoff, Chlor, Methyl, Trifluormethyl, Trifluormethoxy, Difluormethylthio oder Cyano bedeuten, wobei von den letzteren Substituenten Wasserstoff, Chlor, Triflourmethyl oder Triflourmethoxy bevorzugt sind.

Herausragende Verbindungen der Formel (I) sind diejenigen, in denenh $R_1$ Aethyl, $R_2$ Wasserstoff, $R_3$ eine Methoxy- oder Aethoxygruppe, X Trifluormethyl oder Trifluormethoxy und Y und Z Wasserstoff bedeuten.

Speziell bevorzugte Verbindungen sind:

2-(4'-Phenoxyphenyl)-3-methoxycarbonyl-5-methyl-5,6-dihydro-4-pyron,
2-(4'-Phenoxyphenyl)-3-methoxycarbonyl-6-methyl-5,6-dihydro-4-pyron.
2-(4'-Phenoxyphenyl)-3-methoxycarbonyl-5,6-dimethyl-5,6-dihydro-4-pyron (cis-trans-Gemisch),
2-(4'-[4"-Chlorphenoxy]-phenyl)-3-äthoxycarbonyl-5-äthyl-5,6-dihydro-4-pyron,
2-(4'-[4"-Chlorphenoxy]-phenyl)-3-äthoxycarbonyl-5,6-dimethyl-5,6-dihydro-4-pyron (trans),
2-(4'-[4"-Chlorphenoxy]-phenyl)-3-äthoxycarbonyl-5,6-dimethyl-5,6-dihydro-4-pyron (cis), 2-(4'-[4"-Chlorphenoxy]-phenyl)-3-äthoxycarbonyl-6-methyl-5,6-dihydro-4-pyron,
2-(4'-[2",4"-Dichlorphenoxy]-phenyl)-3-äthoxycarbonyl-5-methyl-5,6-dihydro-4-pyron,
2-(4'-[2",4"-Dichlorphenoxy]-phenyl)-3-äthoxycirbonyl-5,6-dimethyl-5,6-dihydro-4-pyron (trans),
2-(4'-[2",4"-Dichlorphenoxy]-phenyl)-3-äthoxycarbonyl-5,6-dimethyl-5,6-dihydro-4-pyron (cis),
2-(4'-[2",4"-Dichlorphenoxy]-phenyl)-3-äthoxycarbonyl-5-äthyl-5,6-dihydro-4-pyron,
2-(4'-[2",4"-Dichlorphenoxy]-phenyl)-3-äthoxycarbonyl-6-äthyl-5,6-dihydro-4-pyron,
2-(4'-[3"-Trifluormethyl-phenoxy]-phenyl)-3-methoxycarbonyl-5,6-dimethyl-5,6-dihydro-4-pyron (cis-trans-Gemisch),
2-(4'-[3"-Trifluormethyl-phenoxy]-phenyl)-3-methoxycarbonyl-5-äthyl-5,6-dihydro-4-pyron,
2-(4'-[3"-Trifluormethyl-phenoxy]-phenyl)-3-methoxycarbonyl-5-methyl-5,6-dihydro-4-pyron,
2-(4'-[4"-Trifluormethyl-phenoxy]-phenyl)-3-methoxycarbonyl-5,6-dimethyl-5,6-dihydro-4-pyron (trans),
2-(4'-[4"-Trifluormethyl-phenoxy]-phenyl)-3-methoxycarbonyl-5,6-dimethyl-5,6-dihydro-4-pyron (cis),
2-(4'-[2"-Chlor-4"-trifluormethyl-phenoxy]-phenyl)-3-methoxycarbonyl-5-äthyl-5,6-dihydro-4-pyron,
2-(4'-[2"-Chlor-4"-trifluormethyl-phenoxy]-phenyl)-3-methoxycarbonyl-6-äthyl-5,6-dihydro-4-pyron,
2-(4'-[2"-Chlor-4"-trifluormethyl-phenoxy]-phenyl)-3-methoxycarbonyl-5-methyl-5,6-dihydro-4-pyron,
2-(4'-[2"-Chlor-4"-trifluormethyl-phenoxy]-phenyl)-3-methoxycarbonyl-5,6-dimethyl-5,6-dihydro-4-pyron (trans),
2-(4'-[2"Chlor-4"-trifluormethyl-phenoxy]-phenyl)-3-methoxycarbonyl-5,6-dimethyl-5,6-dihydro-4-pyron (cis),
2-(4'-[2"-Chlor-4"-trifluormethyl-phenoxy]-phenyl)-3-äthoxycarbonyl-5,6-dimethyl-5,6-dihydro-4-pyron (trans),
2-(4'-[2"-Chlor-4"-trifluormethyl-phenoxy]-phenyl)-3-äthoxycarbonyl-5,6-dimethyl-5,6-dihydro-4-pyron (cis),
2-(4'-[2"-Chlor-4"-trifluormethyl-phenoxyl]-phenyl)-3-äthoxycarbonyl-6-methyl-5,6-dihydro-4-pyron,
2-(4'-[2"-Chlor-4"-trifluormethyl-phenoxy]-phenyl)-3-äthoxycarbonyl-6-äthyl-5,6-dihydro-4-pyron,
2-(4'-[2"-Chlor-4"-trifluormethyl-phenoxy]-phenyl)-3-äthoxycarbonyl-5-äthyl-5,6-dihydro-4-pyron,
2-(4'-[3"-Chlorphenoxy]-phenyl)-3-methoxycarbonyl-5-äthyl-5,6-dihydro-4-pyron,
2-(4'-[3"-Chlorphenoxy]-phenyl)-3-methoxycarbonyl-5,6-dimethyl-5,6-dihydro-4-pyron (cis-trans-Gemisch),
2-(4'-[3"-Trifluormethyl-phenoxy]-phenyl)-3-äthoxycarbonyl-5-äthyl-5,6-dihydro-4-pyron,
2-(4'-[3",5"-Di-{trifluormethyl}-phenoxy]-phenyl)-3-methoxycarbonyl-5-äthyl-5,6-dihydro-4-pyron,
2-(4'[3",5"-Dichlorphenoxy]-phenyl)-3-methoxycarbonyl-5-äthyl-5,6-dihydro-4-pyron,
2-(4'-áA3",5"-Dichlorphenoxy]-phenyl)-3-methoxycarbonyl-5,6-dimethyl-5,6-dihydro-4-pyron (cis-trans-Gemisch),
2-(4'-[",5"-Di-{trifluormethyl}-phenoxy]-phenyl)-3-methoxycarbonyl-5,6-dimethyl-5,6-dihydro-4-pyron (cis-trans-Gemisch),
2-(4'-[3"-Chlorphenoxy]-phenyl)-3-methoxycarbonyl-6-äthyl-5,6-dihydro-4-pyron,
2-(4'-[3",5"-Dichlorphenoxy]-phenyl)-3-methoxycarbonyl-6-äthyl-5,6-dihydro-4-pyron,
2-(4'-[3",5"-Di-{trifluormethyl}-henoxy]-phenyl)-3-methoxycarbonyl-6-äthyl-5,6-dihydro-4-pyron,
2-(4'-[3"-Trifluormethyl-phenoxy]-henyl)-3-methoxycarbonyl-6-äthyl-5,6-dihydro-4-pyron,
2-(4'-[3"-Triflourmetnyl-phenoxy]-phenyl)-3-methoxycarbonyl-5-iso-propyl-5,6-dihydro-4-pyron,

Die Herstellung von Verbindungen der Formel 1 crfolgt analog dem in Bull. Soc. Chim. de France (196S), 255-298, beschriebenen Verfahren, indem man

a) eine Verbindung der Formel (II)

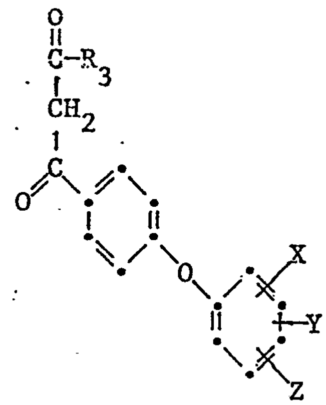 (II);

worin $R_3$, x, y und z die obije Bedentung haben in einem inerten Lösungsmittel mit einer Verbindung der Formel (III)

$Mg(OR')_2$ III

worin R' eine geradkettige oder verzweigte $C_1$-$C_4$-Alkylgruppe bedeutet, umsetz und

b) das erhaltene Reaktionsprodukt in einem inerten Lösungsmittel mit einer Verbindung der Formel (V)

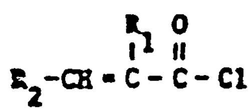 (V),

worin $R_1$ und $R_2$ die obije Bedeutung haben umsetzt und

c) das erhaltene produkt mit einer alkoholischen Lösung einer starken Säure cyclisiert und, wenn $R_3$ in der Formel (I) für Hydroxy steht, den erhaltenen Ester verseift, oder gegebenenfalls in dem erhaltenen Ester der Formel (I) den Rest $R_3$ durch Umesterung in einen anderen, unter Formel (I) definierten Rest $R_3$ überführt.

Die unter a) und b) beschriebenen Umsetzungen werden jeweils in einem inerten Lösungsmittel durchgeführt. Als geeignete Lösungsmittel kommen beispielsweise Benzol, Toluol, Xylol, Aether, Tetrahydrofuran oder Dioxan in Betracht.

Die Umsetzung einer Verbindung der Formel (II) mit einer Verbindung der Formel III erfolgt zweckmässigerweise bei einer Temperatur im Bereich von 0 bis 150°C, die Umsetzung des unter a) erhaltenen Reaktionsproduktes mit einer Verbindung der Formel (V) bei einer Temperatur in Bereich von -10°C bis Raumtemperatur.

Das in der unter a) beschriebenen Umsetzung verwendete Magnesiumalkoholat der Formel (III) kann in situ durch Umsetzung von Magnesium mit einem entsprechenden Alkohol in Gegenwart von $CCl_4$ hergestellt werden.

Die unter c) beschriebene Cyclisierung lässt sich mit einer alkoholischen Lösung einer katalytischen Menge einer starken Säure am Rückfluss durchführen, beispielsweise mit einer alkoholischen Lösung von Chlorwasserstoffsäure, die auch in situ durch Umsetzung von Acetylchlorid mit Alkohol erhältlich ist.

Die Ueberführung eines Restes $R_3$ in einen anderen, unter Formel (I) definierten Rest $R_3$ durch Umesterung kann in an sich bekannter Weise durchgeführt werden, indem man eine Verbindung der Formel (I) mit einem entsprechenden Alkohol in Gegenwart einer starken Säure versetzt.

Die Herstellung von Verbindungen der Formel (I) wird durch die folgenden Beispiele näher erläutert.

**Herstellungsbeispiele für Wirkstoffe**

**Beispiel 1**

2-(4'-[3"-Chlorphenoxy]-phenyl)-3-methoxycarbonyl-5-äthyl-5,6-dihydro-4-pyron

a) 10 ml abs. Methanol und 1 ml $CCl_4$ werden in 50 ml abs. Aether gelöst und zu der Lösung 1,1 g Magnesiumspäne gegeben. Bei einsetzender $H_2$-Entwicklung werden 13,7 g 4-(3'-Chlorphenoxy)-benzoylessigsäuremethylester, gelöst in wenig Aether, hinzugetropft. Die Umsetzung zu 2-[4'-(3"-Chlorphenoxy)-benzoyl]-2-(methoxymagnesium)-essigsäuremethylester erfolgt umgehend unter beträchtlicher Wärmetönung. Zu der erhaltenen goldgelben Lösung werden 100 ml abs. Toluol zugefügt. Anschliessend destilliert man den Aether sowie überschüssiges Methanol ab.

b) Die gemäss a) erhaltene Lösung von 2-[4'-(3"-Chlorphenoxy)-benzoyl]-2-(methoxymagnesium)-essigsäuremethylester in Toluol wird auf 5°C gekühlt. Anschliessend werden 5,5 g 2-Aethylacrylsäurechlorid zugetropft. Man lässt zwei Stunden bei Raumtemperatur ausreagieren und fügt 50 ml abs. Acetonitril zu. Die erhaltene homogene Lösung wird auf Eis/konz. $H_2SO_4$ gegossen und 15 Min. gerührt. Man extrahiert mit Aether, wäscht nacheinander mit 2N $H_2SO_4$, gesättigter $NaHCO_3$-Lösung, gesattigter NaCl-Lösung, trocknet und dampft ein. Man erhält ein viskoses, rotbraunes Oel.

c) Das gemäss b) erhaltene viskose Oel wird in 200 ml Methanol gelöst. Man versetzt mit 1 ml Acetylchlorid und kocht die Lösung über Nacht am Rückfluss. Anschliessend dampft man ein, nimmt mit Aether auf, behandelt mit Aktivkohle und dampft nochmals ein. Das erhaltene viskose Oel wird im Fetroläther/Aether-Gemisch zur Kristallisation gebracht. Man erhält 7,4 g (42,5% d. Th.) 2-(4'-[3ü-Chlorphenoxy]-phenyl)-3-methoxycarbonyl-5-äthyl-5,6-dihydro-4-pyron. Smp. 99-101°C.

**Beispiel 2**

2-(4'-[4"-Chlorphenoxy]-phenyl)-3-äthoxycarbonyl-5,6-dimethyl-5,6-dihydro-4-pyron

a) Analog Beispiel 1 wird aus 14 g 4-(4'-Chlorphenoxy)-benzoylessig-säureäthylester, 1,2 g Magnesiumspänen und 10 ml Aethanol eine Lösung von 2-[4'-(4"-Chlorphenoxy)-benzoyl]-2-(äthoxymagnesium)-essigsaureäthylester in Toluol hergestellt.

b) Unter Kühlung auf -5°C werden 5,7 g 2,3-Dimethylacrylsäurechlorid zu der gemäss a) erhaltenen Lösung von 2-[4'-(4"-Chlorphenoxy)-benzoyl]-2-(äthoxymagnesium)-essigsäureäthylester in Toluol zugetropft. Man lässt 2 Stunden bei Raumtemperatur ausreagieren und versetzt mit 50 ml abs. Acetonitril. Die erhaltene homogene Lösung wird auf Bis/konz. $H_2SO_4$ gegossen und 15 Min. gerührt. Man extrahiert mit Aether und wäscht nacheinander mit 2N $H_2SO_4$, gesättigter $NaHCO_3$-Lösung und gesättigter NaCl-Lösung, trocknet und dampft ein. Man erhält ein viskoses Oel.

c) Das gemäss b) erhaltene viskose Oel wird in 200 ml Aethanol und 1 ml Acetylchlorid über Nacht am Rückfluss zyklisiert. Die Lösung wird eingedampft und an 1100 g Kieselgel in Aether/Hexan 1:1 chromatographiert. Man isoliert 4,5 g trans-2-(4'-[4"-Chlorphenoxy]-phenyl)-3-äthoxycarbonyl-5,6-dimethyl-5,6-dihydro-4-pyron (Smp. 94-96°C, Rf (Aether/ Hexan 1:1) ca. 0,3), sowie 1,2 g cis-2-(4'-[4"-Chlorphenoxy]-phenyl)-3-äthoxycarbonyl-5,6-dimethyl-5,6-dihydro-4-pyron (Smp.88-90°C, Rf (Aether/Hexan 1:1) ca. 0,2). Die Strukturzuordnung erfolgte auf der Basis der unterschiedlichen Kopplungskonstanten $J_{CH(5)-CH(6)}$ im NMR-Spektrum.

[$^J$CH(5)-CH(6)$^{(cis)}$: 3 Hz; $^J$CH(5)-CH(6)$^{(trans)}$: 12 Hz]

Auf analoge Weise können auch folgende Verbindungen der Formel (I) hergestellt werden:

**Tabelle 1**

Verbindungen der Formel (I)

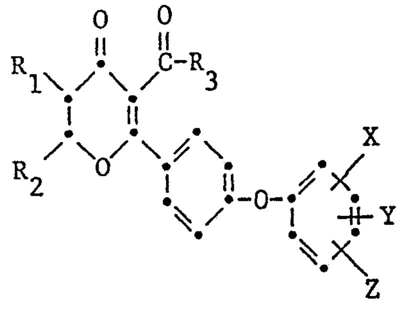

c/t = cis-/trans-Isomerengemisch
c = cis-Isomer
t = trans-Isomer

Tabelle 1

| Nr. | $R_1$ | $R_2$ | $R_3$ | X | Y | Z | Smp °C (Konstitution) | Konfiguration |
|---|---|---|---|---|---|---|---|---|
| 1 | $CH_3$ | H | $OCH_3$ | H | H | H | (viskos) | - |
| 2 | H | $CH_3$ | $OCH_3$ | H | H | H | 86-90 | - |
| 3 | $CH_3$ | $CH_3$ | $OCH_3$ | H | H | H | (viskos) | c/t |
| 4 | $C_2H_5$ | H | $OC_2H_5$ | H | 4-Cl | H | 101-102 | - |
| 5 | $CH_3$ | $CH_3$ | $OC_2H_5$ | H | 4-Cl | H | 94-96 | t |
| 6 | $CH_3$ | $CH_3$ | $OC_2H_5$ | H | 4-Cl | H | 88-90 | c |
| 7 | H | $CH_3$ | $OC_2H_5$ | H | 4-Cl | H | 96-97 | - |
| 8 | $CH_3$ | H | $OC_2H_5$ | 2-Cl | 4-Cl | H | 92-95 | - |
| 9 | $CH_3$ | $CH_3$ | $OC_2H_5$ | 2-Cl | 4-Cl | H | 94-97 | t |
| 10 | $CH_3$ | $CH_3$ | $OC_2H_5$ | 2-Cl | 4-Cl | H | 92-95 | c |
| 11 | $C_2H_5$ | H | $OC_2H_5$ | 2-Cl | 4-Cl | H | 103-104 | - |
| 12 | H | $C_2H_5$ | $OC_2H_5$ | 2-Cl | 4-Cl | H | 95-98 | - |
| 13 | $CH_3$ | $CH_3$ | $OCH_3$ | $3-CF_3$ | H | H | 115-130 | c/t |

Fortsetzung Tabelle 1

| Nr. | $R_1$ | $R_2$ | $R_3$ | X | Y | Z | Smp °C (Konstitution) | Konfiguration |
|---|---|---|---|---|---|---|---|---|
| 14 | $C_2H_5$ | H | $OCH_3$ | 3-$CF_3$ | H | H | 77-78 | - |
| 15 | $CH_3$ | H | $OCH_3$ | 3-$CF_3$ | H | H | 82-83 | - |
| 16 | $CH_3$ | $CH_3$ | $OCH_3$ | H | 4-$CF_3$ | H | 88-91 | t |
| 17 | $CH_3$ | $CH_3$ | $OCH_3$ | H | 4-$CF_3$ | H | (wachsartig) | c |
| 18 | $C_2H_5$ | H | $OCH_3$ | 2-Cl | 4-$CF_3$ | H | (viskos) | - |
| 19 | H | $C_2H_5$ | $OCH_3$ | 2-Cl | 4-$CF_3$ | H | (viskos) | - |
| 20 | $CH_3$ | H | $OCH_3$ | 2-Cl | 4-$CF_3$ | H | (viskos) | - |
| 21 | $CH_3$ | $CH_3$ | $OCH_3$ | 2-Cl | 4-$CF_3$ | H | (viskos) | t |
| 22 | $CH_3$ | $CH_3$ | $OCH_3$ | 2-Cl | 4-$CF_3$ | H | (viskos) | c |
| 23 | $CH_3$ | $CH_3$ | $OC_2H_5$ | 2-Cl | 4-$CF_3$ | H | (viskos) | t |
| 24 | $CH_3$ | $CH_3$ | $OC_2H_5$ | 2-Cl | 4-$CF_3$ | H | (viskos) | c |
| 25 | H | $CH_3$ | $OC_2H_5$ | 2-Cl | 4-$CF_3$ | H | (viskos) | - |
| 26 | H | $C_2H_5$ | $OC_2H_5$ | 2-Cl | 4-$CF_3$ | H | (viskos) | - |

Fortsetzung Tabelle 1

| Nr. | $R_1$ | $R_2$ | $R_3$ | X | Y | Z | Smp °C (Konstitution) | Konfiguration |
|---|---|---|---|---|---|---|---|---|
| 27 | $C_2H_5$ | H | $OC_2H_5$ | 2-Cl | 4-$CF_3$ | H | (viskos) | - |
| 28 | $C_2H_5$ | H | $OCH_3$ | 3-Cl | H | H | 98-101 | - |
| 29 | $CH_3$ | $CH_3$ | $OCH_3$ | 3-Cl | H | H | 123-128 | c/t |
| 30 | $C_2H_5$ | H | $OC_2H_5$ | 3-$CF_3$ | H | H | 63-65/80-81 | - |
| 31 | $C_2H_5$ | H | $OCH_3$ | 3-$CF_3$ | H | 5-$CF_3$ | 107-109 | - |
| 32 | $C_2H_5$ | H | $OCH_3$ | 3-Cl | H | 5-Cl | 95-97 | - |
| 33 | $CH_3$ | $CH_3$ | $OCH_3$ | 3-Cl | H | 5-Cl | 110-117 | c/t |
| 34 | $CH_3$ | $CH_3$ | $OCH_3$ | 3-$CF_3$ | H | 5-$CF_3$ | 133-135 | c/t |
| 35 | H | $C_2H_5$ | $OCH_3$ | 3-Cl | H | H | 74-76 | - |
| 36 | H | $C_2H_5$ | $OCH_3$ | 3-Cl | H | 5-Cl | 135-138 | - |
| 37 | H | $C_2H_5$ | $OCH_3$ | 3-$CF_3$ | H | 5-$CF_3$ | 112-115 | - |
| 38 | H | $C_2H_5$ | $OCH_3$ | 3-$CF_3$ | H | H | 87-89 | - |
| 39 | $C_3H_7$iso | H | $OCH_3$ | 3-$CF_3$ | H | H | 74-75 | - |

Fortsetzung Tabelle 1

| Nr. | $R_1$ | $R_2$ | $R_3$ | X | Y | Z | Smp. °C (Konstitution) | Konfiguration |
|---|---|---|---|---|---|---|---|---|
| 40 | $C_2H_5$ | H | $OC_3H_7$iso | 3-$CF_3$ | H | H | - | - |
| 41 | $C_2H_5$ | H | $OC_4H_9$tert | 3-$CF_3$ | H | H | - | - |
| 42 | $C_2H_5$ | H | $OCH_2CH_2Cl$ | 3-$CF_3$ | H | H | - | - |
| 43 | $C_2H_5$ | H | $OCH_2CH{=}CH_2$ | 3-$CF_3$ | H | H | - | - |
| 44 | $C_2H_5$ | H | $OCH_2C{\equiv}CH$ | 3-$CF_3$ | H | H | - | - |
| 45 | $C_2H_5$ | H | $OCH_2CH_2OCH_3$ | 3-$CF_3$ | H | H | - | - |
| 46 | —$(CH_2)_3$— | | $OCH_2CF_3$ | 3-$CF_3$ | H | H | - | - |
| 47 | —$(CH_2)_4$— | | $OC_2H_5$ | 3-$CF_3$ | H | H | 107 - 111 | c/t |
| 48 | $C_3H_7$iso | H | $OC_2H_5$ | 3-$CF_3$ | H | H | - | - |
| 49 | $C_4H_9$tert | H | $OC_2H_5$ | 3-$CF_3$ | H | H | - | - |
| 50 | $C_2H_5$ | H | $OC_2H_5$ | 3-Cl | H | H | 78-80 | - |
| 51 | $C_2H_5$ | H | $OCH_2CCl_3$ | 3-Cl | H | H | - | - |
| 52 | —$(CH_2)_3$— | | $OC_2H_5$ | 3-Cl | H | H | - | - |

Fortsetzung Tabelle 1

| Nr. | $R_1$ | $R_2$ | $R_3$ | X | Y | Z | Smp. °C (Konstitution) | Konfiguration |
|---|---|---|---|---|---|---|---|---|
| 53 | $C_2H_5$ | H | $OC_2H_5$ | 3-F | H | H | 79-80 | - |
| 54 | H | $C_2H_5$ | $OC_2H_5$ | 3-F | H | H | - | - |
| 55 | $C_4H_9$tert | H | $OC_2H_5$ | 3-F | H | H | - | - |
| 56 | $C_2H_5$ | H | $OC_2H_5$ | 3-Br | H | H | 84-85 | - |
| 57 | H | $C_2H_5$ | $OCH_2CH{=}CH_2$ | 3-Br | H | H | - | - |
| 58 | —$(CH_2)_4$— | | $OC_2H_5$ | 3-Br | H | H | 133-139 | c/t |
| 59 | $C_2H_5$ | H | $OC_2H_5$ | 3-J | H | H | 93-96 | - |
| 60 | H | $C_2H_5$ | $OC_2H_5$ | 3-J | H | H | | |
| 61 | $C_2H_5$ | H | $OC_2H_5$ | 3-$OCF_3$ | H | H | 54-56 | - |
| 62 | H | $C_2H_5$ | $OC_2H_5$ | 3-$OCF_3$ | H | H | (viskos) | - |
| 63 | $C_4H_9$tert | H | $OC_2H_5$ | 3-$OCF_3$ | H | H | - | - |
| 64 | $C_3H_7$iso | H | $OC_2H_5$ | 3-$OCF_3$ | H | H | - | - |
| 65 | $C_2H_5$ | H | $OCH_2CH_2Cl$ | 3-$OCF_3$ | H | H | - | - |

Fortsetzung Tabelle 1

| Nr. | $R_1$ | $R_2$ | $R_3$ | X | Y | Z | Smp. °C (Konstitution) | Konfiguration |
|---|---|---|---|---|---|---|---|---|
| 66 | $C_2H_5$ | H | $OC_2H_5$ | 3-$OCHF_2$ | H | H | (viskos) | - |
| 67 | H | $C_2H_5$ | $OC_2H_5$ | 3-$OCHF_2$ | H | H | - | - |
| 68 | $C_2H_5$ | H | $OCH_2OCH_3$ | 3-$OCHF_2$ | H | H | - | - |
| 69 | $CH_3$ | $CH_3$ | $OC_3H_7$iso | 3-$OCHF_2$ | H | H | - | - |
| 70 | —$(CH_2)_3$— | | $OCH_2C{\equiv}CH$ | 3-$OCHF_2$ | H | H | - | - |
| 71 | $C_2H_5$ | H | $OC_2H_5$ | 3-$SCH_3$ | H | H | 59-61 | - |
| 72 | H | $C_2H_5$ | $OC_2H_5$ | 3-$SCH_3$ | H | H | - | - |
| 73 | $C_2H_5$ | H | $OC_3H_7$n | 3-$SCH_3$ | H | H | - | - |
| 74 | $C_2H_5$ | H | $OC_2H_5$ | 3-$SOCH_3$ | H | H | - | - |
| 75 | H | $C_2H_5$ | $OC_2H_5$ | 3-$SOCH_3$ | H | H | - | - |
| 76 | $C_2H_5$ | H | $OC_2H_5$ | 3-$SO_2CH_3$ | H | H | (viskos) | - |
| 77 | $C_3H_7$iso | H | $OC_2H_5$ | 3-$SO_2CH_3$ | H | H | - | - |
| 78 | $C_2H_5$ | H | $OC_2H_5$ | 3-$OCF_2CHF_2$ | H | H | (viskos) | - |

Fortsetzung Tabelle 1

| Nr. | $R_1$ | $R_2$ | $R_3$ | X | Y | Z | Smp. °C (Konstitution) | Konfiguration |
|---|---|---|---|---|---|---|---|---|
| 79 | H | $C_2H_5$ | $OC_2H_5$ | $3-OCF_2CHF_2$ | H | H | - | - |
| 80 | $C_3H_7$iso | H | $OC_2H_5$ | $3-OCF_2CHF_2$ | H | H | - | - |
| 81 | $C_2H_5$ | H | $OCH_2CH_2Cl$ | $3-OCF_2CHF_2$ | H | H | - | - |
| 82 | $C_2H_5$ | H | $OC_2H_5$ | $3-SCHF_2$ | H | H | (viskos) | - |
| 83 | $-(CH_2)_4-$ | | $OC_2H_5$ | $3-SCHF_2$ | H | H | - | - |
| 84 | $C_2H_5$ | H | $OC_2H_5$ | $3-CH_3$ | H | H | 69-70 | - |
| 85 | H | $C_2H_5$ | $OC_2H_5$ | $3-CH_3$ | H | H | - | - |
| 86 | $C_2H_5$ | H | $OC_2H_5$ | $3-OCH_3$ | H | H | 64-65 | - |
| 87 | $C_2H_5$ | H | $OCH_2CCl_3$ | $3-OCH_3$ | H | H | - | - |
| 88 | H | $C_2H_5$ | $OC_2H_5$ | $3-OCH_3$ | H | H | - | - |
| 89 | $C_2H_5$ | H | $OCH_3$ | $3-C(O)OCH_3$ | H | H | - | - |
| 90 | H | $C_2H_5$ | $OC_2H_5$ | $3-C(O)OC_2H_5$ | H | H | - | - |
| 91 | $C_2H_5$ | H | $OC_2H_5$ | $3-NO_2$ | H | H | - | - |

Fortsetzung Tabelle 1

| Nr. | $R_1$ | $R_2$ | $R_3$ | X | Y | Z | Smp. °C (Konstitution) | Konfiguration |
|---|---|---|---|---|---|---|---|---|
| 92 | $CH_3$ | $CH_3$ | $OC_2H_5$ | $3-NO_2$ | H | H | - | - |
| 93 | $C_2H_5$ | H | $OC_2H_5$ | $3-NH_2$ | H | H | - | - |
| 94 | $C_2H_5$ | H | $OC_2H_5$ | $3-N(CH_3)_2$ | H | H | 78-79 | - |
| 95 | $C_2H_5$ | H | $OC_2H_5$ | 3-CN | H | H | 75-77 | - |
| 96 | H | $C_2H_5$ | $OCH_2CH_2Cl$ | 3-CN | H | H | - | - |
| 97 | $C_2H_5$ | H | $OC_2H_5$ | $3-NHCH_3$ | H | H | - | - |
| 98 | $C_2H_5$ | H | $OC_2H_5$ | H | $4-SCF_3$ | H | - | - |
| 99 | $C_2H_5$ | H | $OC_2H_5$ | H | $4-SOCF_3$ | H | - | - |
| 100 | $C_2H_5$ | H | $OC_2H_5$ | H | $4-OCHF_2$ | H | - | - |
| 101 | H | $C_2H_5$ | $OC_2H_5$ | H | $4-OCF_3$ | H | - | - |
| 102 | H | $C_2H_5$ | $OC_2H_5$ | H | 4-F | H | - | - |
| 103 | $C_2H_5$ | H | $OC_2H_5$ | H | 4-F | H | - | - |

Fortsetzung Tabelle 1

| Nr. | $R_1$ | $R_2$ | $R_3$ | X | Y | Z | Konfiguration |
|---|---|---|---|---|---|---|---|
| 104 | $C_2H_5$ | H | $OC_2H_5$ | H | 4-J | H | - |
| 105 | $C_2H_5$ | H | $OC_2H_5$ | 2-F | H | H | - |
| 106 | $C_2H_5$ | H | $OC_2H_5$ | 2-Cl | H | H | - |
| 107 | H | $C_2H_5$ | $OC_2H_5$ | 2-Cl | H | H | - |
| 108 | $C_2H_5$ | H | $OC_2H_5$ | 2-Cl | 3-Cl | H | - |
| 109 | $C_2H_5$ | H | $OC_2H_5$ | 3-Cl | 4-Cl | H | - |
| 110 | $C_2H_5$ | H | $OC_2H_5$ | 2-Cl | H | 6-Cl | - |
| 111 | $C_2H_5$ | H | $OC_2H_5$ | $2-CF_3$ | H | H | - |
| 112 | $C_4H_9$tert | H | $OC_2H_5$ | $2-CF_3$ | H | H | - |
| 113 | $C_2H_5$ | H | $OC_2H_5$ | H | 4-CN | H | - |
| 114 | H | $C_2H_5$ | $OC_2H_5$ | H | 4-CN | H | - |
| 115 | $C_2H_5$ | H | $OC_2H_5$ | 2-Br | H | H | - |
| 116 | $C_2H_5$ | H | $OC_2H_5$ | 2-Br | 3-Br | H | - |

Fortsetzung Tabelle 1

| Nr. | $R_1$ | $R_2$ | $R_3$ | X | Y | Z | Smp. °C (Konstitution) | Konfiguration |
|---|---|---|---|---|---|---|---|---|
| 117 | H | $C_2H_5$ | $OC_2H_5$ | 2-$OCH_3$ | H | H | - | - |
| 118 | $C_2H_5$ | H | $OC_2H_5$ | H | 4-$OCH_3$ | H | - | - |
| 119 | $C_2H_5$ | H | $OC_2H_5$ | H | 4-$SCH_3$ | H | - | - |
| 120 | H | $C_2H_5$ | $OC_2H_5$ | H | 4-$CH_3$ | H | - | - |
| 121 | $C_2H_5$ | H | $OC_2H_5$ | 3-$C_2H_5$ | H | H | - | - |
| 122 | $C_2H_5$ | H | $OC_2H_5$ | H | 4-$C_2H_5$ | H | - | - |
| 123 | $C_3H_7$n | H | $OCH_3$ | 3-$CF_3$ | H | H | - | - |
| 124 | $C_4H_9$sek | H | $OCH_3$ | 3-$CF_3$ | H | H | - | - |
| 125 | $C_4H_9$n | H | $OC_2H_5$ | 3-$CF_3$ | H | H | 85-87 | - |
| 126 | $C_2H_5$ | H | $OC_2H_5$ | H | H | H | 86-88 | - |
| 127 | $CH_3$ | H | $OC_2H_5$ | 3-$CF_3$ | H | H | 96-97 | - |
| 128 | H | $C_2H_5$ | $OC_2H_5$ | 3-$CF_3$ | H | H | 70-72 | - |
| 129 | H | $C_2H_5$ | $OC_2H_5$ | 3-Br | H | H | 68-70 | - |

Fortsetzung Tabelle 1

| Nr. | $R_1$ | $R_2$ | $R_3$ | X | Y | Z | Smp. °C (Konstitution) | Konfiguration |
|---|---|---|---|---|---|---|---|---|
| 130 | $CH_3$ | $CH_3$ | $OC_2H_5$ | 3-$CF_3$ | H | H | 103-108 | c/t |
| 131 | $CH_3$ | $CH_3$ | $OC_2H_5$ | 3-Br | H | H | 129-134 | c/t |
| 132 | $CH_3$ | $CH_3$ | $OC_2H_5$ | 3-$OCF_3$ | H | H | 70-80 | c/t |
| 133 | $C_2H_5$ | H | $OC_3H_7$n | 3-$CF_3$ | H | H | 64-67 | - |
| 134 | $C_2H_5$ | H | $OC_4H_9$n | 3-$CF_3$ | H | H | 47-49 | - |
| 135 | $C_4H_9$iso | H | $OC_2H_5$ | 3-Br | H | H | 74-75 | - |
| 136 | $C_4H_9$iso | H | $OC_2H_5$ | 3-$CF_3$ | H | H | 82-83 | - |
| 137 | $C_4H_9$iso | H | $OC_2H_5$ | 3-$OCF_3$ | H | H | 47-49 | - |
| 138 | $C_4H_9$n | H | $OC_2H_5$ | 3-Br | H | H | 76-78 | - |
| 139 | $C_2H_5$ | H | OH | 3-$CF_3$ | H | H | (viskos) | - |
| 140 | $C_4H_9$n | H | $OC_2H_5$ | 3-$OCF_3$ | H | H | 81-82 | - |
| 141 | $-(CH_2)_4-$ | | $OC_2H_5$ | 3-$OCF_3$ | H | H | 82-86 | c/t |
| 142 | $-(CH_2)_3-$ | | $OC_2H_5$ | 3-$CF_3$ | H | H | 111-112 | c |
| 143 | $-(CH_2)_3-$ | | $OC_2H_5$ | 3-$OCF_3$ | H | H | 81-83 | c |
| 144 | $-(CH_2)_3-$ | | $OC_2H_5$ | 3-Br | H | H | 110-111 | c |
| 145 | $-(CH_2)_4-$ | | $OC_2H_5$ | 3-$OCF_3$ | H | H | 92-93 | c |

**Tabelle 2:**

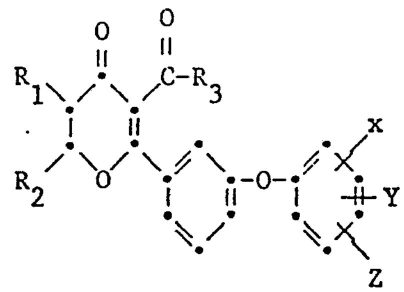

| Nr. | $R_1$ | $R_2$ | $R_3$ | X | Y | Z | Konstitution | Konfiguration |
|---|---|---|---|---|---|---|---|---|
| 146 | $C_2H_5$ | H | $OCH_3$ | 3-Cl | H | H | viskos | - |

Die Ausgangsprodukte der Formel (II) sind zum Teil bekannt (Gruppe A), zum Teil neu (Gruppe B) und lassen sich nach bekannten Verfahren oder auf analoge Weise herstellen.

Gruppe A besteht aus Verbindungen der Formel (II), in denen der Phenoxyrest in p-Stellung steht, $R_3$ $C_1$-$C_4$-Alkoxy ist und zwei der Substituenten X, Y und Z für Wasserstoff stehen und der dritte Substituent Wasserstoff, Fluor, Chlor, Brom oder Trifluormethyl ist.

Gruppe B besteht aus Verbindungen der Formel (II), in denen $R_3$ $C_1$-$C_4$-Alkoxy, Allyloxy, Proparyloxy, 2,2,2-Trichloräthoxy, 2,2,2-Trifluoräthoxy, 2-Chloräthoxy oder $C_1$-$C_4$-Alkoxyl, $C_1$-$C_4$ alkoxy mit maximal 5 C-Atomen und X, Y und Z unabhängig voneinander Wasserstoff, Halogen, $C_1$-$C_4$-Alkyl, $C_1$-$C_4$-Alkoxy, $C_1$-$C_4$-Halogenalkyl, Trifluormethoxy, Difluormethoxy, 1,1,2,2-Tetraflouräthoxy $S(O)_n$-$C_1$-$C_4$-Alkyl, wobei n für 0, 1 oder 2 steht Trifluormethylthio, Diflourmethylthio, Triflourmethlsulfinyl, $C(O)OR_4$, wobei $R_4$ für Wasserstoff oder $C_1$-$C_4$-Alkyl steht, $NO_2$, CN oder $NR_5R_6$, wobei $R_5$ und $R_6$ unabhängig voneinander für Wasserstoff, $C_1$-$C_4$-Alkyl, $C_1$-$C_4$-Alkylcarbonyl, $C_1$-$C_4$-Halogen, alkylcarbonyl stehen, bedeuten, mit der Massgabe, dass, $R_3$ $C_1$-$C_4$-Alkoxy ist und zwei der Substituenten X, Y und Z für Wasserstoff stehen, der dritte Substituent nicht Wasserstoff, Fluor, Chlor, Brom oder Trifluormethyl bedeutet.

Die Verbindungen der Formel (II), Gruppe B, die speziell für die Herstellung der erfindungsgemässen Verbindungen der Formel als Zwischen produkte entwickelt wurden, bilden den Gegenstand der Teilanmeldung 85105831.3 (= EP-A-164 590)

Ein Verfahren zur Herstellung von C-(4'-Chlorphenoxy)-benzoylessig-säureäthylester durch Umsetzung von 4-(4'-Chlorphenoxy)-benzoesäure mit Thionylchlorid und Natrium-Acetylessigsäureäthylester und anschliessende Deacetylierung wird in Arzneimittelforschung 30 (1), Nr. 3 (1980), 454-459, beschrieben.

In der DE-A- 2 436 012 werden 4-Phenoxy-benzoylessigsäure und -alkylester, in denen die Phenoxygruppe unsubstituiert oder durch ein Fluor-, Chlor- oder Bronatom oder eine Trifluormethylgruppe substituiert ist. beschrieben. Die Alkylester sind durch Friedel-Crafts-Acylierung der entsprechenden Diphenyläther mit Malonesterchloriden, und die entsprechenden Säuren durch anschliessende Verseifung erhältlich.

Ausser nach den vorstehend bereits angeführten Verfahren lassen sich Verbindungen der Formel (II) auch analog dem in J. Amer. Chem. Soc. 70 (I9C8). 3356-3360, beschriebenen Verfahren durch Umsetzung von Phenoxyphenylderivaten mit Malonsäuremethylesterchlorid herstellen.

Ferner kann die Herstellung von Verbindungen der Formel (II), in denen $R_3$ für $C_1$-$C_4$ -Alkoxy steht analog dem in Tetrahedron 20 (1964) 1625-1632, beschriebenen Verfahren gemäss folgender schematischer Darstellung erfolgen:

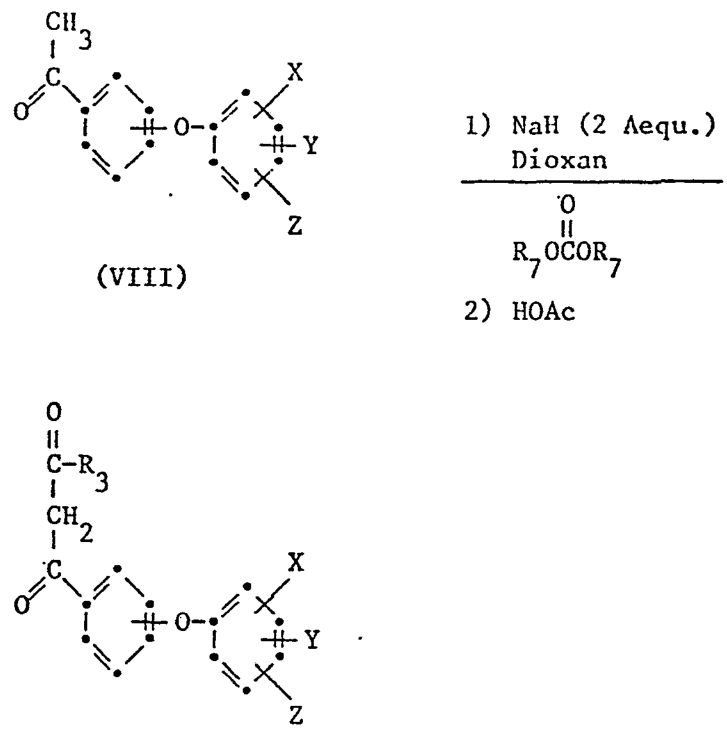


(VIII)

(II)

In den vorstehenden Formeln haben X Y und Z die für Formel (II) angegebenen Bedeutungen und $R_7$ steht für $C_1$-$C_4$-Alkyl.

Die Verbindungen der Formel (VIII) sind bekannt oder können analog bekannten Methoden hergestellt werden (s. z.B. US-A- 4.125.729; BE-A- 639.727 [Ref. in Chem. Abst. 62 (1965), 14581h]).

Im folgenden Beispiel wird die Herstellung von Verbindungen der Formel II analog dem letztgenannten Verfahren näher erläutert.

**Herstellungsbeispiel für Ausgangsstoffe**

**Beispiel 3**

4-(3'-Trifluormethyl-phenoxy)-benzoylessigsäuremethyl-ester

Zu einer Mischung von 29 g NaH (2 Aequ.; 55%ige Oeldispersion, dreimal mit Toluol gewaschen) und 54 g Dimethylcarbonat in 400 ml Dioxan werden bei 85°C 84,2 g 4-(3'-Trifluormethylphenoxy)-acetophenon gelöst in 160 ml Dioxin, hinzugetroprt, wobei sofort Wasserstoffentwicklung erfolgt. Nich dem Abklingen der Reaktion wird noch 2 Stunden bei 85°C gerührt. Unter Eiskühlung werden 100 ml Essigsäure zugetropft. Die erhaltene Paste wird mit 400 ml Aether sowie ca.200 g Alox 1 (Aluminiumoxid (Alumina) Woelm, Aktivitätsstufe 1) neutral versetzt. Das Gemisch wird filtriert. Das Filtrat wird eingedampft, in Aceton gelöst, nacheinander mit Wasser, gesättigter $NaHCO_3$-Lösung und gesättigter NaCl-Lösung gewaschen, getrocknet und eingedampft. Das erhaltene viskose Produkt kann direkt für die Herstellung von un 160°C destilliert oder aus Hexan umkristallisiert (Smp. 50-55°C). Die Ausbeute an gereinigtem Produkt beträgt ca. 65 g (65% d. Th.).

Auf analoge Weise können auch folgende Verbindungen der Formel (II) hergestellt werden:

**Tabelle 3**
Gruppe A

| Nr. | $R_3$ | X | Y | Z | Smp °C (Konstitution) |
|---|---|---|---|---|---|
| A-1 | $OCH_3$ | H | H | H | 48-52 |
| A-2 | $OCH_3$ | 3-$CF_3$ | H | H | 50-55 |
| A-3 | $OCH_3$ | H | 4-$CF_3$ | H | (viskoses Oel) |
| A-4 | $OC_2H_5$ | 3-$CF_3$ | H | H | (viskos) |
| A-5 | $OCH_3$ | 3-Cl | H | H | (viskos) |
| A-6 | $OC_2H_5$ | H | 4-Cl | H | - |
| A-7 | $OC_3H_7$iso | 3-$CF_3$ | H | H | (viskos) |
| A-8 | $OC_4H_9$tert | 3-$CF_3$ | H | H | - |
| A-9 | $OC_2H_5$ | 3-Cl | H | H | (viskos) |
| A-10 | $OC_2H_5$ | 3-F | H | H | (viskos) |
| A-11 | $OC_2H_5$ | 3-Br | H | H | (viskos) |
| A-12 | $OC_2H_5$ | H | 4-F | H | - |
| A-13 | $OC_2H_5$ | 2-F | H | H | - |
| A-14 | $OC_2H_5$ | 2-Cl | H | H | - |
| A-15 | $OC_2H_5$ | 2-$CF_3$ | H | H | - |
| A-16 | $OC_2H_5$ | 2-Br | H | H | - |
| A-17 | $OC_3H_7$n | 3-$CF_3$ | H | H | (viskos) |
| A-18 | $OC_4H_9$n | 3-$CF_3$ | H | H | (viskos) |

Zur Applikation als Herbizide werden die Verbindungen der Formel (I) in unveränderter Form oder vorzugsweise zusammen mit den in der Formlierungstechnik üblichen Hilfsmitteln eingesetzt und werden daher z.B. zu Emulsionskonzentraten, streichfähigen Pasten, direkt versprühbaren oder verdünnberen Lösungen, verdünnten Emulsionen, Spritzpulveren, löslichen Pulvern, Stäubemitteln, Granulaten, auch Verkapselungen in z.B. polymeren Stoffen in beknnter Weise verarbeitct. Die Anwendungsverfahren wie Versprühen, Vernebeln, Verstäuben, Verstreuen, Bestreichen,oder Giessen werden gleich wie die Art der Mittel den angestrebten Zielen und den gegebenen Verhältnissen entsprechend gewählt.

Die Formulierungen d.h. die den Wirkstoff der Formel 1 und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder

Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus können eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel 1 nichtionogene, kation- und/oder anionaktive Tenside mit guten Emulgier', Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen genannt, die z.B. aus Kokosnuss- oder Talgöl gewonnen werden können. Ferner sind auch die Fettsäuremethyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Caoder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylcnoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an polypropylenglykol, Aethylendiaminopolypropylenglykol und Alkylpolypropylenglykolmit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole Ricinusölpolyglykoläther Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschricben:

"Mc Cutcheon's Detergents and Emulsifiers Annual" MC Publishing Corp., Ringwood New Jersey, 1980 Sisely and Wood, "Encyclopedia of Surface Active Agents", Chemical Publishing Co., Inc. New York, 1980.

Die agrochemischen Zubereitungen enthalten in der Regel 0,1 bis 99%, insbesondere 0,1 bis 95%, Wirkstoff der Formel I, 99,9 bis 1% insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Formulierungsbeispiele für flüssige Wirkstoffe der Formel (I) (%=Gewichtsprozent) 4. Emulsions-Konzentrate** a) b) c)

Wirkstoff aus Tabelle 1 oder 2 25g 40% 50%

Ca-Dodecylbenzolsulfonat 5% 8ü 6%
Ricinusölpolyäthylenglykoläther (36 Mol AeO) 5% -
Tributylphenolpolyäthylenglykoläther (30 Mol AeO) - 12% 4%
Cyclohexanon - 15% 20%
Xylolgemisch 65% 25% 20%

Aus solchen Konzentraten können durch Verdünnen mit Wasser-Emulsionen jeder gewünschten Konzentration hergestellt werden.

**5. Lösungen** a) b) c) d)
Wirkstoff aus Tabelle 1 oder 2 80% 10% 5% 95%
Acthylenglykolmonomethyläther 20% - -
Polyäthylenglykol MG 400 - 70% -
N-Methyl-2-pyrrolidon - 20ü -
Epoxydiertes Kokosnussöl - - 1% 5%
Benzin (Siedegrenzen 160-190,C) - - 94%

Die Lösungen sind zur Anwendung in Form kleinster Tropfen geeignet.

**6. Granulate** a) b)
Wirkstoff aus Tabelle 1 oder 2 5% 10%
Kaolin 94%
Hochdisperse Kieselsäure 1%
Ataapulgit - 90%

Der Wirkstoff wird in Methylenchlorid gelöst, auf den Träger aufgesprüht und das Lösungsmittel anschliessend im Vakuum abgedampft.

**7. Stäubemittel** a) b)
Wirkstoff aus Tabelle 1 oder 2 2% 5%
Hochdisperse Kieselsäure 1% 5%
Talkum 97%
Kaolin - 90%

Durch inniges Vermischen der Trägerstoffe mit dem Wirkstoff erhält mangebrauchsfertige Stäubemittel.

**Formulierungsbeispiele für feste Wirkstoffe der Formel (I) (% = Gewichtsprozent)**

**8. Spritzpulver** a) b) c)
Wirkstoff aus Tabelle 1 oder 2 25% 50% 75%
Na-Ligninsulfonat 5% 5%
Na-Laurylsulfat 3% - 5%
Na-Diisobutylnaphthalinsulfonat - 6% 10%
Octylphenolpolyäthylenglykoläter (7-8 Mol AeO) - 2%
Hochdisperse Kieselsäure 5% 10% 10%
Kiolin 62% 27%

Der Wirkstoff wird mit den Zusatzstoffen gut vermischt und in einergeeigneten Mühle gut vermahlen. Man erhält Spritzpulver, die sich mit Wasser zu Suspensionen jeder gewünschten Konzentration verdünnen lassen.

**9. Emulsions-Konzentrat**
Wirkstoff aus Tabelle 1 oder 2 10%
Octylphenolpolyäthylenglykoläther (4-5 Mol AeO) 3%
Ca-Dodecylbenzolsulfonat
Ricinusölpolyglykoläther (35 Mol AeO) 4%
Cyclohexanon 30%
Xylolgemisch 50%

Aus diesem Konzentrat können durch Verdünnen mit Wasser Emulsionen jeder gewünschten Konzentration hergestellt werden.

**10. Stäubemittel** a) b)
Wirkstoff aus Tabelle 1 oder 2 5% 8%
Talkum 95%
Kaolin - 92%

Man erhält anwendungsfertige Stäubemittel, indem der Wirkstoff mit den-Träger vermischt und auf einer geeigneten Mühle vermahlen wird.

**11. Extruder-Granulat**
Wirkstoff aus Tabelle 1 oder 2 10%
Na-Ligninsulfonat 2%
Carboxymethylcellulose 1%
Kaolin 87%

Der Wirkstoff wird mit den Zusatzstoffen vermischt, vermahlen und mit Wasser angefeuchtet. Dieses Gemisch wird extrudiert und anschliessend im Luftstrom getrocknet.

**12. Umhüllungs-Granulat**
Wirkstoff aus Tabelle 1 oder 2 3%
Polyäthylenglykol (M G 200) 3%
Kaolin 94%

Der fein gemahlene Wirkstoff wird in einem Mischer auf das mit Folyäthylenglykol angefeuchtete Kaolin gleichmässig aufgetragen. Auf diese WWeise erhält man staubfreie Umhüllungs-Granulate.

**13. Suspensions-Konzentrat**

Wirkstoff aus Tabelle 1 oder 2 40%
Aethylenglykol 10%
Nonylphenolpolyäthylenglykoläther (15 Mol AeO) 6%
N-Ligninsulfonat 10%
Carboxymethylcellulose 1%
37%ige wässrige Formaldehyd-Lösung 0,2%
Silikonöl in Form einer 75%igen wässrigen Emulsion 0,8%
Wasser 32%

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Die Verbindungen der Formel (I) weisen ausgezeichnete herbizide Eigenschaften auf. Sie sind zur Bekämpfung sowohl monokotyler wie dikotyler Yflanzen geeignet, und zwar sowohl bei der Anwendung im Vorauflaufverfahren (preemergent) wie im Nachauflaufverfahren (postemergent). Besonders vorteilhaft lassen sich die Verbindungen der Forme (I) zioder sie enthaltende Mittel zur selektiven Bekämpfung von Unkräutern in Getreidekulturen einsetzen.

Wegen ihrer vorteilhaften Eigenschaften besonders erwähnenswerte Verbindungen der Formel I sind 2-(4'-[3"-Trifluormethyl-phenoxy]-phenyl)-3-methoxycarbonyl-5-äthyl-5,6-dihydro-4-pyron und 2-(4'-[3"-Trifluormethyl-phenoxy]-phenyl)-3-äthoxycarbonyl-5-äthyl-5,6-dihydro-4-pyron.

Bei stereoisomeren Verbindungen (wie beispielssveise Verbindungen 5 und 6) wirkt die cis-Verbindung stärker als die trans-Verbindung.

2-Phenyl-5,6-dihydro-4-pyrone mit herbiziden Bigenschaften sind aus der DE-A- 2 910 283 bekannt. Sie weisen am Phenylrest Substituenten der Gruppe Halogen, $C_1$-$C_4$-Alkyl und $C_1$-$C_4$-Alkoxy auf und sind somit strukturell deutlich verschieden von den erfindungsgemässen Verbindungen der Formel I.

**Biologische Beispiele**

**Beispiel 14**

Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrate hergestellt werden können, behandelt. Es wurden Konzentrationen von 4 kg Wirksubstanz/Hektar angewendet. Die Saatschalen werden im Gewächshaus bei 22-25°C und 50-70% relativer Luftfeuchtigkeit gehalten und der Versuch nach 3 Wochen ausgewertet und die Resultate nach folgender Notenskala bonitiert:

1 = Pflanzen nicht gekemit oder total abgestorben
2-3 = sehr starke Wirkung
4-6 = mittlere Wirkung
7-8 = geringe Wirkung
9 = keine Wirkung (wie unbehandelte Kontrolle)

Pre-emergente Wirkung

Aufwandmenge: 4 kg Wirksubstanz/Hektar

| Verb. Nr. | Avena | Setaria | Sinapis | Stellaria |
|---|---|---|---|---|
| 2 | 7 | 3 | 2 | 3 |
| 5 | 7 | 4 | 5 | 3 |
| 6 | 5 | 2 | 1 | 1 |
| 7 | 7 | 5 | 4 | 1 |
| 10 | 8 | 5 | 3 | 4 |
| 13 | 7 | 2 | 2 | 1 |
| 14 | 6 | 1 | 1 | 1 |
| 15 | 6 | 2 | 3 | 1 |
| 17 | 7 | 2 | 3 | 1 |
| 19 | 7 | 4 | 4 | 2 |
| 28 | 6 | 2 | 2 | 1 |
| 29 | 9 | 4 | 2 | 1 |
| 30 | 5 | 1 | 1 | 1 |
| 35 | 7 | 1 | 1 | 1 |
| 37 | 8 | 5 | 2 | 1 |
| 38 | 6 | 1 | 1 | 1 |
| 50 | 9 | 2 | 4 | 2 |
| 56 | 6 | 1 | 1 | 1 |
| 59 | 7 | 4 | 5 | 3 |
| 61 | 7 | 1 | 1 | 1 |
| 62 | 8 | 1 | 1 | 1 |
| 66 | 6 | 3 | 1 | 2 |
| 78 | 7 | 3 | 2 | 3 |
| 82 | 9 | 1 | 5 | 1 |
| 86 | 7 | 5 | 3 | 3 |
| 127 | 6 | 1 | 1 | 2 |
| 128 | 7 | 1 | 1 | 2 |
| 129 | 7 | 3 | 1 | 3 |
| 130 | 6 | 3 | 2 | 1 |
| 132 | 6 | 1 | 1 | 1 |
| 133 | 9 | 2 | 1 | 1 |
| 134 | 8 | 5 | 3 | 1 |

**Beispiel 15**

Post-emergente Herbizid-Wirkung (Kontaktherbizid)

Eine Anzahl Unkräuter, sowohl monokotyle wie dikotyle, wurden nach dem Auflaufen (in 4- bis 6-Blattstadium) mit einer wässrigen Wirkstoffdispersion in einer Dosierung von 4 kg Wirksubstanz pro Hektar auf die pflanzen gespritzt und diese bei 24'-26°C und 45-60% relativer Luftfeuchtigkeit gehalten. Mindestens 15 Tage nach der Behandlung wird der Versuch ausgewertet und das Ergebnis nach derselben Notenskala wie im pre-emergenten Versuch (Beispiel 14) bonitiert.

Post-emergente Wirkung

Aufwandmenge: 4 kg Wirksubstanz/Hektar

| Verb. Nr. | Avena | Setaria | Lolium | Solanum | Sinapis | Stellaria | Phaseolus |
|---|---|---|---|---|---|---|---|
| 5 | 6 | 6 | 7 | 2 | 2 | 6 | 5 |
| 6 | 6 | 5 | 5 | 2 | 2 | 5 | 4 |
| 7 | 6 | 6 | 7 | 2 | 3 | 6 | 7 |
| 10 | 6 | 4 | 6 | 2 | 3 | 5 | 5 |
| 13 | 6 | 4 | 5 | 2 | 3 | 4 | 4 |
| 14 | 6 | 4 | 4 | 1 | 3 | 4 | 3 |
| 15 | 6 | 4 | 6 | 2 | 2 | 4 | 3 |
| 17 | 6 | 4 | 5 | 2 | 3 | 4 | 4 |
| 19 | 8 | 6 | 6 | 1 | 3 | 5 | 6 |
| 26 | 8 | 6 | 6 | 2 | 3 | 4 | 6 |
| 28 | 8 | 6 | 6 | 3 | 3 | 6 | 3 |
| 29 | 8 | 6 | 6 | 3 | 3 | 6 | 6 |
| 30 | 5 | 5 | 4 | 4 | 2 | 4 | 4 |
| 33 | 7 | 9 | 9 | 4 | 4 | 5 | 6 |
| 35 | 7 | 6 | 4 | 2 | 4 | 4 | 5 |
| 37 | 8 | 7 | 9 | 3 | 4 | 6 | 6 |
| 38 | 6 | 6 | 6 | 3 | 4 | 5 | 6 |
| 50 | 6 | 4 | 5 | 3 | 3 | 3 | 3 |
| 53 | 6 | 5 | 5 | 3 | 3 | 4 | 4 |
| 56 | 8 | 7 | 6 | 2 | 2 | 4 | 5 |
| 59 | 6 | 8 | 5 | 2 | 2 | 5 | 4 |
| 61 | 6 | 3 | 4 | 2 | 3 | 4 | 4 |
| 62 | 7 | 4 | 5 | 2 | 2 | 5 | 6 |
| 66 | 6 | 6 | 3 | 2 | 2 | 5 | 3 |
| 71 | 6 | 6 | 6 | 3 | 3 | 3 | 3 |
| 76 | 7 | 6 | 6 | 3 | 3 | 3 | 4 |
| 78 | 6 | 6 | 4 | 2 | 3 | 4 | 5 |
| 82 | 7 | 7 | 6 | 3 | 3 | 3 | 4 |
| 84 | 7 | 6 | 3 | 3 | 2 | 5 | 4 |
| 86 | 6 | 6 | 3 | 2 | 2 | 4 | 3 |
| 95 | 7 | 7 | 3 | 2 | 2 | 3 | 4 |
| 127 | 6 | 6 | 6 | 3 | 4 | 6 | 5 |
| 128 | 6 | 3 | 4 | 2 | 3 | 4 | 4 |
| 129 | 7 | 3 | 4 | 2 | 3 | 4 | 5 |
| 132 | 6 | 4 | 6 | 3 | 3 | 4 | 4 |
| 133 | 6 | 6 | 6 | 4 | 3 | 5 | 5 |

**Patentansprüche**

1. Verbindungen der Formel (I)

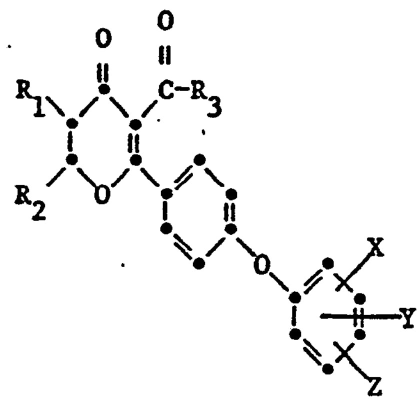

(I),

worin $R_1$ und $R_2$ unabhängig voneinander eine $C_1$-$C_4$-Alkylgruppe oder eine der beiden Substituenten auch Wasserstoff, oder $R_1$ und $R_2$ gemeinsam eine $C_2$-$C_6$-Alkylenbrück, $R_3$ $C_1$-$C_4$-Alllyloxy, Propapgyloxy, 2,2,2-Trichloräthoxy, 2,2,2-Triflouräthoxy, 2-Chloräthoxy, $C_1$-$C_4$-Alkoxy-$C_1$-$C_4$-alkoxy mit maximal 5 Kohlenstoffatomen, Hydroxy oder Salze dieser Carbonsäuren und X, Y und Z unbhängig vineinander Wasserstoff, Halogen, $C_1$-$C_4$-Alkyl, $C_1$-$C_4$-Alkoxy, $C_1$-$C_4$-Halogenalkyl, Triflourmethoxy, Difluormethoxy, 1,1,2.2-Tetrafluoräthoxy, -S(O) -$C_1$-$C_4$-Alkyl, wobei n für 0,1 · oder 2 steht, Trifluormethylthio, Difluormethylthio, Trifluoremethylsulfinyl C(O)$OR_4$, wobei $R_4$ für Wasserstoff oder $C_1$-$C_4$-Alkyl steht, $NO_2$, CN oder $NR_5R_6$, wobei $R_5$ und $R_6$ unabhängig voneinander für Wasserstoff, $C_1$-$C_4$-Alkyl, $C_1$-$C_4$-Alkylcarbonyl oder $C_1$-$C_4$-Halogenalkylcarbonyl stehen, bedeuten, unter Einschluss ihrer Säureaditionssalze.

2. Verbindungen der Formel (I) nach Anspruch 1, dadurch gekennzeichnet. dass $R_1$ für $C_1$-$C_4$-Alkyl und $R_2$ für Methyl oder Aethyl oder einer der Substituenten $R_1$ und $R_2$ auch für Wasserstoff steht oder $R_1$ und $R_2$ zusammen eine -$(CH_2)_3$- oder -$(CH_2)_4$-Gruppe bilden, $R_3$ für $C_1$-$C_4$-Alkoxy. Helches geradkettig oder verzweigt sein kann, Methoxymethoxy oder 2-(Methoxy)-äthoxy steht. einer der Substituenten X, Y und Z für Wasserstoff steht und die beiden anderen Substituenten unabhängig voneinander Wasserstoff, Chlor, Brom, Jod, Fluor, Methyl, Aethyl, Methoxy, Trifluormethyl, Trifluormethoxy, Difluormethoxy, 1,1,2,2-Tetrafluoräthoxy Trofluormethythio, Diflourmethylthio, Triflourmethylsulfinyl, Methoxycarbonyl, Aethoxycarbonyl, Nitro, Cyano, Amino, Methylamino oder Dimethylamino bedeuten.

3. Verbindungen der Formel (I) nach Anspruch 2, dadurch gekennzeichnet, dass einer der Substituenten $R_1$ und $R_2$ für Wasserstoff und der andere für Methyl oder Aethyl oder $R_1$ und $R_2$ beide für Methyl stehen, $R_3$ eine Methoxy- oder Aethoxygruppe darstellt. einer der Substituenten X, Y und Z für Wasserstoff steht und die beiden anderen Substituenten unabhängig voneinander Wasserstoff, Chlor, Methyl, Trifluormethyl. Trifluormethoxy, Difluormethylthio oder Cyano bedeuten.

4. Verbindungen der Formel (I) nach Anspruch 3, dadurch gekennzeichnet, daß einer der Substituenten X, Y und Z für Wasserstoff steht und die beiden anderen Substituenten unabhängig voneinander Wasserstoff. Chlor, Trifluormethyl oder Trifluormethoxy bedeuten.

5. Verbindungen der Formel (I) nach Anspruch 4, dadurch gekennzeichnet, dass $R_1$ Aethyl, $R_2$ Wasserstoff, $R_3$ eine Methoxy- oder Aethoxygruppe. X Trifluormethyl oder Trifluormethoxy und Y und Z Wasserstoff bedeuten.

6. 2-(4'-[3"-Trifluormethyl-phenoxy]-phenyl)-3-methoxycarbonyl-5-äthyl-5,6-dihydro-4-pyron.

7. 2-(4'-[3"-Trifluormethyl-Phenoxy]-phenyl)-3-äthoxycarbonyl-5-äthyl-5,6-dihydro-C-Pyron.

8. Verfahren zur Herstellung von Verbindungen der Formel (I) dadurch gekennzeichnet, dass man

a) eine Verbindung der Formel (II)

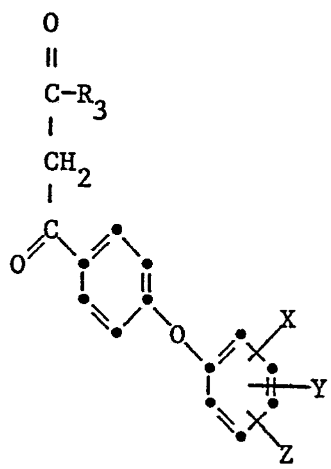

(II),

worin $R_3$, X, Y und Z die unter Anspruch 1 angegebene Bedeutung haben, in einem inerten Lösungsmittel mit einer Verbindung der Formel (III)

$Mg(OR')_2$ III

worin R' eine geradkettige oder verzweigte $C_1$-$C_4$-Alkylgruppe bedeutet, umsetzt und

b) das erhaltene Reaktionsprodukt in einem inerten Lösungsmittel mit einer Verbindung der Formel (V)

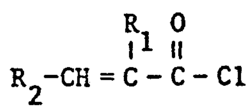

(V),

worin $R_1$ und $R_2$ unter Anspruch 1 angegebene bedeutung haben, umsetzt und

c) das erhaltene Produkt mit einer alkoholischen Lösung einer starken Säure cyclisiert und, wenn $R_3$ in der Formel (I) für Hydroxy steht, den erhaltenen Ester verseift, oder gegebenenfalls in dem erhaltenen Ester der Formel (I) den Rest $R_3$ durch Umesterung in einen anderen, unter Formel 1 definierten Rest $R_3$ überführt.

9. Herbizides Mittel, dadurch gekennzeichnet, dass es als aktive Komponente mindestens eine Verbindung der Formel (I) nach Anspruch 1 enthält.

10. Herbizides Mittel nach Anspruch 9, dadurch gekennzeichnet, dass es 0,1 bis 99 Gewichtsprozent einer Verbindung der Formel (I) und 1 bis 99,9 Gewichtsprozcnt eincs festen oder flüssigen Zusatzstoffes, darunter 0 bis 25 Gewichtsprozent eines Tensides, enthält.

11. Herbizides Mittel nach Anspruch 10, dadurch gekennzeichnet, dass es 0,1 bis 95 Gewichtsprozent einer Verbindung der Formel (I), 5 bis 99,8 Gewichtsprozent eines festen oder flüssigen Zusatzstoffes und 0,1 bis 25 Gewichtsprozent eines Tensides, enthält.

12. Verwendung einer Verbindung der Formel (I) nach Anspruch 1 zur Bekämpfung von Unkräutern.

13. Verwendung nach Anspruch 12 zur selektiven Bekämpfung von Unkräutern in Getreidekulturen.

14. Verfahren zur Bekämpfung von Unkräutern, dadurch gekennzeichnet, dass man eine wirksame Menge einer Verbindung der Formel (I) nach Anspruch 1 auf die Schadpflanze, deren Pflanzenteile oder deren Standort appliziert.

**Claims**

1. A compound of the formula (I)

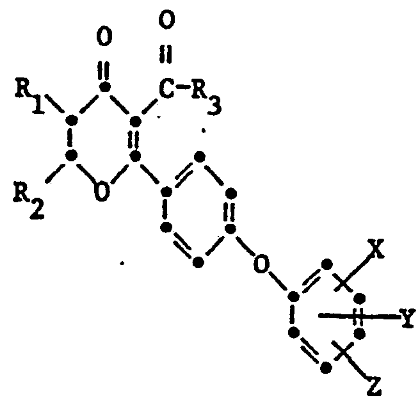

I

wherein $R_1$ and $R_2$ are each independently a $C_1$-$C_4$alkyl group, or one of the two substituents is also hydrogen, or $R_1$ and $R_2$ jointly form a $C_2$-$C_6$alkylene bridge, $R_3$ is $C_1$-$C_4$alkoxy, allyloxy, propargyloxy, 2,2,2-trichloroethoxy, 2,2,2-trifluoroethoxy, 2-chloroethoxy, $C_1$-$C_4$alkoxy-$C_1$-$C_4$alkoxy containing not more than 5 carbon atoms, or hydroxyl or salt of a resultant carboxylic acid, and X, Y and Z are each independently hydrogen, halogen, $C_1$-$C_4$alkyl, $C_1$-$C_4$alkoxy, $C_1$-$C_4$haloalkyl, trifluoromethoxy, difluoromethoxy, 1,1,2,2-tetrafluoroethoxy or -$S(O)_n$-$C_1$-$C_4$alkyl, where n is 0, 1 or 2, or are trifluoromethylthio, difluoromethylthio, trifluoromethylsulfinyl or $C(O)OR_4$, where $R_4$ is hydrogen or $C_1$-$C_4$alkyl, or are $NO_2$, CN or $NR_5R_6$, where $R_5$ and $R_6$ are each independently hydrogen, $C_1$-$C_4$alkyl, $C_1$-$C_4$alkylcarbonyl or $C_1$-$C_4$haloalkylcarbonyl, or an acid addition salt therenf.

2. A compound of the formula (I) according to claim 1, wherein $R_1$ is $C_1$-$C_4$alkyl and $R_2$ is methyl or ethyl, or one of the substituents $R_1$ and $R_2$ is also hydrogen, or $R_1$ and $R_2$ together form a -$(CH_2)_3$- or $(CH_2)_4$- group, $R_3$ is $C_1$-$C_4$alkoxy which may be straight chain or branched, or methoxymethoxy or 2-(methoxy)ethoxy, and one of the substituents X, Y and Z is hydrogen, and the two other substituents are each independently hydrogen, chlorine, bromine, iodine, fluorine, methyl, ethyl, methoxy, trifluoromethyl, trifluoromethoxy, difluoromethoxy, 1,1,2,2-tetrafluoro-ethoxy, methylthio, methylsulfinyl, methylsulfonyl, trifluoromethylthio, difluoromethylthio, trifluoromethylsulfinyl, methoxycarbonyl, ethoxycarbonyl, nitro, cyano, amino, methylamino or dimethylamino.

3. A compound of the formula (I) according to claim 2, wherein one of the substituents $R_1$ and $R_2$ is hydrogen and the other is methyl or ethyl, or $R_1$ and $R_2$ are both methyl, $R_3$ is a methoxy or ethoxy group, and one of the substituents X, Y and Z is hydrogen, and the two other substituents are each independently hydrogen, chlorine, methyl, trifluoromethyl, trifluoromethoxy, difluoromethlythio or cyano.

4. A compound of the formula (I) according to claim 3, wherein one of the substituents X, Y and Z is hydrogen, and the two other substituents are each independently hydrogen, chlorine, trifluoromethyl or trifluoromethoxy.

5. A compound of the formula (I) according to claim 4, wherein $R_1$ is ethyl, $R_2$ is hydrogen, $R_3$ is a methoxy or ethoxy group, X is trifluoromethyl or trifluoromethoxy, and Y and Z are hydrogen.

6. 2-(4'-[3'''-Trifluoromethylphenoxy]phenyl)-3-methoxycarbonyl-5-ethyl-5,6-dihydro-4-pyrone.

7. 2-(4'-[3"-Trifluoromethylphenoxy]phenyl)-3-ethoxycarbonyl-5-ethyl-5,6-dihydro-4-pyrone.

8. A process for the preparation of a compound of the formula (I), which process comprises

a) reacting a compound of the formula (II)

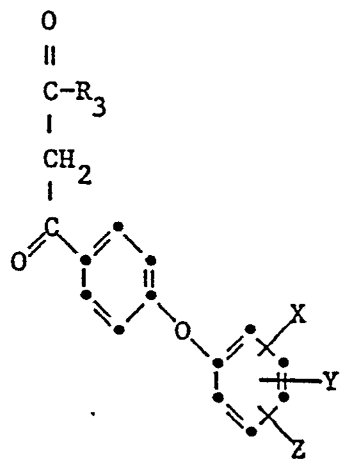

II

wherein $R_3$, X, Y and Z are as defined in claim 1, in an inert solvent, with a compound of the formula (III)
$Mg(OR')_2$ III
wherein R' is a straight chain or branched $C_1$-$C_4$alkyl group;
b) reacting the resultant reaction product, in an inert solvent, with a compound of the formula (V)

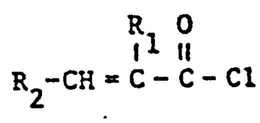

V

wherein $R_1$ and $R_2$ are as defined in claim 1, and

c) cyclising the resultant product with an alcoholic solution of a strong acid, and, if $R_3$ in the formula (I) is hydroxyl, saponifying the ester obtained, or optionally converting in the resultant ester of the formula (I) the group $R_3$, by transesterification, into another group $R_3$ defined under the formula (I).

9. A herbicidal composition containing, as active ingredient, at least one compound of the formula (I) according to claim 1.

10. A herbicidal composition according to claim 9, which contains 0.1 to 99 per cent by weight of a compound of the formula (I), and 1 to 99.9 per cent by weight of a solid or liquid additive, including 0 to 25 per cent by weight of a tenside.

11. A herbicidal composition according to claim 10, which contains 0.1 to 95 per cent by weight of a compound of the formula (I), 5 to 99.8 per cent by weight of a solid or liquid additive, and 0.1 to 25 per cent by weight of a tenside.

12. A method of combating weeds, which method comprises the use of a compound of the formula (I) according to claim 1.

13. A-method according to claim 12 of selectively combating weeds in crops of cereals.

14. A method according to claim 12 of combating weeds, which method comprises applying to the undesirable plants or to parts of the plants or to the locus thereof an effective amount of a compound of the formula (I) according to claim 1.

**Revendications**

1. Composés de formule I

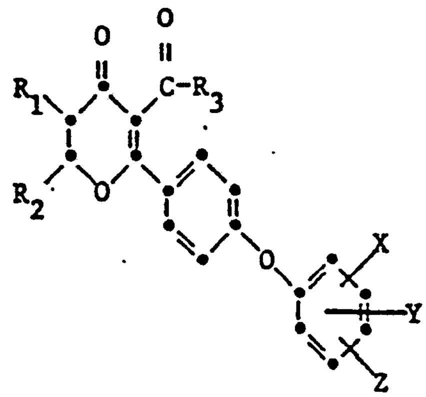

I

dans laquelle $R_1$ et $R_2$ représentent chacun, indédamment l'un de l'autre, un groupe alkyle en C 1-C 4 ou bien l'un des deux symboles représente l'hydrogène, ou bien $R_1$ et $R_2$ forment ensemble un pont alkylène en C 2-C 6, $R_3$ représente un groupe alcoxy en C 1-C 4, allyloxy, propargyloxy, 2,2,2-trichloréthoxy, 2,2,2-trifluoréthoxy, 2-chloréthoxy, (alcoxy en C 1-C 4)-alcoxy en C 1-C 4 contenant au maximum 5 atomes de carbone, hydroxy ou des sels de ces acides carboxyliques, et X, Y et Z représentent chacun, indépendamment les uns des autres, l'hydrogène, un halogène, un groupe alkyle en C 1-C 4, alcoxy en C 1-C 4, halogénoalkyle en C 1-C 4, trifluorométhoxy, difluorométhoxy, 1,1,2,2-tétrafluoréthoxy, $-S(O)_n$-alkyle en C 1-C 4 dans lequel n est égal à 0, 1 ou 2, trifluorométhylthio, difluorométhylthio, trifluorométhylsulfinyle, $C(O)OR_4$ dans lequel $R_4$ représente l'hydrogène ou un groupe alkyle en C 1-C 4, $NO_2$, CN ou $NR_5R_6$ dans lequel $R_5$ et $R_6$ représentent chacun, indépendamment l'un de l'autre, l'hydrogène, un groupe alkyle en C 1-C 4, (alkyle en C 1-C 4)-carbonyle, (halogénoalkyle en C 1-C 4)-carbonyle, y compris leurs sels formés par addition avec des acides.

2. Composés de formule I selon la revendication 1, caractérisés en ce que $R_1$ représente un groupe alkyle en C 1-C 4 et $R_2$ un groupe méthyle ou éthyle ou bien l'un des symboles $R_1$ et $R_2$ représente l'hydrogène ou bien $R_1$ et $R_2$ forment ensemble un groupe $-(CH_2)_3-$ ou $-(CH_2)_4-$, $R_3$ représente un groupe alcoxy en C 1-C 4 à chaîne droite ou ramifiée, méthoxyméthoxy ou 2-(méthoxy)-éthoxy, l'un des symboles X, Y et Z représente l'hydrogène et les deux autres, indépendamment l'un de l'autre, représentent chacun l'hydrogène, le chlore, le brome, l'iode, le fluor, un groupe méthyle, éthyle, méthoxy, trifluorométhyle, trifluorométhoxy, difluorométhoxy, 1,1,2,2-tétrafluoréthoxy, méthylthio, méthylsulfinyle, méthylsulfonyle, trifluorométhylthio, difluorométhylthio, trifluorométhylsulfinyle, méthoxycarbonyle, éthoxycarbonyle, nitro, cyano, amino, méthylamino ou diméthylamino.

3. Composés de formule I selon la revendication 2, caractérisés en ce que l'un des symboles $R_1$ et $R_2$ représente-l'hydrogène et l'autre un groupe méthyle ou éthyle, ou bien $R_1$ et $R_2$ représentent tous deux des groupes méthyle, $R_3$ représente un groupe méthoxy ou éthoxy, l'un des symboles X, Y et Z représente l'hydrogène et les deux autres, indépendamment l'un de l'autre, l'hydrogène, le chlore, un groupe méthyle, trifluorométhyle, trifluorométhoxy, difluorométhylthio ou cyano.

4. Composés de formule I selon la revendication 3, caractérisés en ce que l'un dés symboles X, Y et Z represente l'hydrogène et les deux autres, indépendamment l'un de l'autre, l'hydrogène, le chlore, un groupe trifluorométhyle ou trifluorométhoxy.

5. Composés de formule I selon la revendication 4, caractérisés en ce que $R_1$ représente un groupe éthyle $R_2$ représente l'hydrogène, $R_3$ représente un groupe méthoxy ou éthoxy, X représente un groupe trifluorométhyle ou trifluorométhoxy et Y et Z représentent des atomes d'hydrogène.

6. La 2-(4'-[3"-trifluorométhyl-phénoxy]-phényl)-3-méthoxycarbonyl-5-éthyl-5,6-dihydro-4-pyrone.

7. La 2-(4'-[3"-trifluorométhyl-phénoxy]-phényl)-3-éthoxycarbonyl-5-éthyl-5,6-dihydro-4-pyrone.

8. Procédé de préparation des composés de formule I, caractérisé en ce que:

a) on fait réagir un composé de formule II

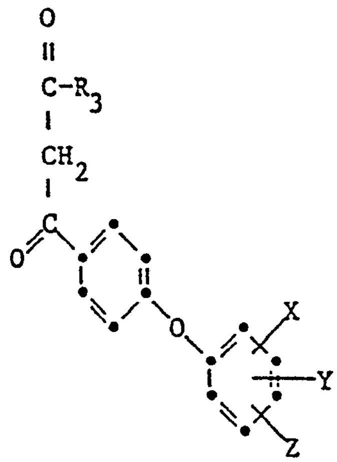

II

dans laquelle $R_3$, X, Y et Z ont les significations indiquées dans la revendication 1, dans un solvant inerte, avec un composé de formule III

$Mg(OR')_2$ III

dans laquelle R' représente un groupe alkyle en C 1-C 4 à chaîne droite ou ramifiée, et

b) on fait réagir le produit obtenu, dans un solvant inerte, avec un composé de formule V

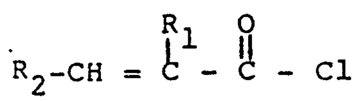

V

dans laquelle $R_1$ et $R_2$ ont les significations indiquées dans la revendication 1, et

c) on cyclise le produit obtenu à l'aide d'une solution alcoolique d'un acide fort et, lorsque $R_3$ de la formule I représente un groupe hydroxy, on saponifie l'ester obtenu, ou bien, le cas échéant, dans l'ester de formule I ainsi obtenu, on convertit le reste $R_3$ par transestérification en un autre reste $R_3$ tel que défini en référence à la formule I.

9. Produit herbicide, caractérisé en ce qu'il contient en tant que composant actif au moins un composé de formule I selon la revendication 1.

10. Produit herbicide selon la revendication 9, caractérisé en ce qu'il contient de 0,1 à 99% en poids d'un composé de formule I et de 1 à 99,9% en poids d'un additif solide ou liquide, dont 0 à 25% en poids d'un agent tensioactif.

11. Produit herbicide selon la revendication 10, caractérisé en ce qu'il contient de 0,1 à 95% en poids d'un composé de formule I, de 5 à 99,8% en poids d'un additif solide ou liquide et de 0,1 à 25% en poids d'un agent tensioactif.

12. Utilisation d'un composé de formule I selon la revendication 1 dans la lutte contre les mauvaises herbes.

13. Utilisation selon la revendication 12, dans la lutte sélective contre les mauvaises herbes dans les cultures de céréales.

14. Procédé pour combattre les mauvaises herbes, caractérisé en ce que l'on applique sur les végétaux nuisibles, des parties de ces végétaux ou leur habitat, une quantité efficace d'un composé de formule I selon la revendication 1.